# EUROPEAN PATENT APPLICATION

(11) **EP 3 438 140 A1**
(43) Date of publication of application: **06.02.2019**
(21) Application number: 17774282.2
(22) Date of filing: 14.03.2017
(51) Int. Cl.: C08F 22/40, H01L 21/56, H01L 23/29, H01L 23/31

(54) **THERMOSETTING COMPOUND**

(30) Priority: 30.03.2016 JP 2016068665
(71) Applicant: Daicel Corporation, Osaka-shi, Osaka 530-0011 (JP)
(72) Inventor: NAKATANI, Kouji, Himeji-shi Hyogo 671-1283 (JP); INOUE, Keizo, Himeji-shi Hyogo 671-1283 (JP)
(74) Representative: Grünecker Patent- und Rechtsanwälte PartG mbB
(86) International application number: PCT/JP2017/010175
(87) International publication number: WO 2017/169738

(57) **Abstract**

Provided is a thermosetting compound that has satisfactory solvent solubility and can be rapidly cured by a heat treatment to form a cured product having extreme heat resistance. The thermosetting compound according to the present invention is represented by following Formula (1). In Formula (1), R¹ and R² each independently represent a thermosetting group; D¹ and D² are each, identically or differently, selected from a single bond and a linkage group; Ar¹, Ar², and Ar³ are each, identically or differently, selected from a divalent aromatic hydrocarbon group and a divalent group including two or more aromatic hydrocarbons bonded to each other via any one selected from a single bond, a divalent aliphatic hydrocarbon group, and a divalent alicyclic hydrocarbon group; and E represents, independently on each occurrence, an ester bond.

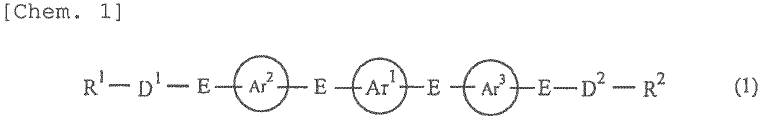

## Description

### Technical Field

The present invention relates to thermosetting compounds and cured products of the thermosetting compounds. This application claims priority to Japanese Patent Application No. 2016-068665, filed to Japan on March 30, 2016, the entire contents of which are incorporated herein by reference.

### Background Art

With recent technological development in the fields of semiconductors and circuit boards, demands have been made to provide thermosetting compounds that have satisfactory solvent solubility and can form cured products having extreme heat resistance, namely, cured products having high decomposition temperatures and maintaining high hardness even in a high-temperature environment at 250°C or higher.

Patent Literature (PTL) 1 to 3 describe thermosetting compounds which are liquid crystal oligomers containing, at an end thereof, a thermosetting group selected from phenylethynyl, phenylmaleimid-N-yl, and nadimid-N-yl.

Disadvantageously, however, curing of the thermosetting compounds requires heating at a high temperature of 350°C or higher for a long time, and such high-temperature heating for curing impairs the semiconductors and circuit boards. Also disadvantageously, the cured products of the thermosetting compounds have 5% weight loss temperatures (T_{d5}) lower than 450°C and may be decomposed by the high-temperature heating for curing.

PTL 4 discloses a thermosetting polyester composition resulting from melt blending a liquid-crystal polyester and a compound with each other, where the liquid-crystal polyester contains at least one of hydroxy and acyloxy at molecular chain ends, and the compound contains a thermosetting group in combination with a functional group reactive with at least one of hydroxy and acyloxy. The literature describes that the thermosetting polyester composition is thermally cured at a temperature of 250°C or lower and gives a cured product that has excellent heat resistance.

### Citation List

### Patent Literature

PTL 1: Japanese Unexamined Patent Application Publication (Translation of PCT Application) No. 2004-509190
PTL 2: U.S. Pat. No. 6939940
PTL 3: U.S. Pat. No. 7507784
PTL 4: PCT International Publication Number WO2014/050850

### Summary of Invention

### Technical Problem

The thermosetting polyester composition, however, has unsatisfactory solvent solubility. Disadvantageously, the composition has poor curability, requires a long time (for example, about 6 hours) for thermal curing even when combined with a crosslinker or a curing accelerator, and thereby has poor workability. Also disadvantageously, the composition, if heated at a high temperature of 300°C or higher so as to be cured within a short time, is liable to decompose.

Accordingly, the present invention has an object to provide a thermosetting compound that has satisfactory solvent solubility and can be rapidly cured by a heat treatment to give a cured product having extreme heat resistance (extremely high heat resistance).

The present invention has another object to provide a thermosetting composition that can be rapidly cured by a heat treatment to give a cured product having extreme heat resistance.

The present invention has yet another object to provide a method for producing a semiconductor device using the thermosetting composition.

The present invention has still another object to provide a semiconductor device produced by the production method.

### Solution to Problem

After intensive investigations to achieve the objects, the inventors of the present invention found that a compound represented by following Formula (1) has satisfactory solvent solubility and can be rapidly cured by heating to give a cured product having extreme heat resistance, specifically, a cured product having a 5% weight loss temperature (T_{d5}) of 450°C or higher and a storage modulus (E') of 1 GPa or more at 250°C. The present invention has been made on the basis of these findings.

Specifically, the present invention provides, in an aspect, a thermosetting compound represented by Formula (1): 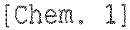 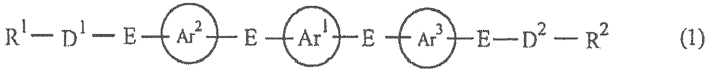 wherein R¹ and R² each independently represent a thermosetting group; D¹ and D² are each, identically or differently, selected from a single bond and a linkage group; Ar¹, Ar², and Ar³ are each, identically or differently, selected from a divalent aromatic hydrocarbon group and a divalent group including two or more aromatic hydrocarbons bonded to each other via any one selected from a single bond, a divalent aliphatic hydrocarbon group, and a divalent alicyclic hydrocarbon group; and E represents, identically or differently on each occurrence, an ester bond selected from -(C=O)O- and -O(C=O)-.

In the thermosetting compound, R¹ and R² in Formula (1) may each be, identically or differently, a thermosetting group selected from the class consisting of phenylethynyl, styryl, maleimidyl, nadimidyl, biphenylenyl, ethynyl, isocyanato, cyanato, nitrile, phthalonitrile, cyclobenzobutenyl, benzoxazinyl, oxetanyl, and vinyl.

The thermosetting compound may have thermotropic liquid crystallinity.

The present invention provides, in another aspect, a thermosetting composition including the thermosetting compound.

The present invention provides, in yet another aspect, a cured product of the thermosetting composition.

The present invention provides, in still another aspect, a method for producing a semiconductor device. The method includes the step of encapsulating a semiconductor element using the thermosetting composition.

In addition and advantageously, the present invention provides a semiconductor device. The semiconductor device structurally includes a semiconductor element, and the cured product covering the semiconductor element.

Specifically, the present invention relates to the followings:
(1) A thermosetting compound represented by Formula (1), wherein R¹ and R² each independently represent a thermosetting group; D¹ and D² are each, identically or differently, selected from a single bond and a linkage group; Ar¹, Ar², and Ar³ are each, identically or differently, selected from a divalent aromatic hydrocarbon group and a divalent group including two or more aromatic hydrocarbons bonded to each other via any one selected from a single bond, a divalent aliphatic hydrocarbon group, and a divalent alicyclic hydrocarbon group; and E represents, identically or differently on each occurrence, an ester bond selected from -(C=O)O- and -O(C=O)-.
(2) The thermosetting compound according to (1), wherein R¹ and R² in Formula (1) are each, identically or differently, a thermosetting group selected from the class consisting of phenylethynyl, styryl, maleimidyl, nadimidyl, biphenylenyl, ethynyl, isocyanato, cyanato, nitrile, phthalonitrile, cyclobenzobutenyl, benzoxazinyl, oxetanyl, and vinyl.
(3) The thermosetting compound according to (1), wherein R¹ and R² in Formula (1) are each, identically or differently, a thermosetting group selected from the class consisting of phenylethynyl, styryl, maleimidyl, nadimidyl, biphenylenyl, phthalonitrile, cyclobenzobutenyl, and benzoxazinyl.
(4) The thermosetting compound according to any one of (1) to (3), wherein Ar¹, Ar², and Ar³ in Formula (1) are each, identically or differently, a group selected from groups represented by Formulae (a1) to (a4).
(5) The thermosetting compound according to any one of (1) to (4), wherein D¹ and D² in Formula (1) are each independently selected from a single bond and a divalent hydrocarbon group (preferably a divalent aromatic hydrocarbon group, particularly preferably a C₆-C₁₄ divalent aromatic hydrocarbon group, and most preferably a group selected from the groups represented by Formulae (a1) to (a4)).
(6) The thermosetting compound according to any one of (1) to (5), wherein the R¹-D¹ group and the R²-D² group in Formula (1) are each, identically or differently, a group selected from the class consisting of nadimidyl, biphenylenyl, ethynyl, isocyanato, cyanato, nitrile, phthalonitrile, cyclobenzobutenyl, benzoxazinyl, oxetanyl, vinyl, methylmaleimidyl, cinnamoyl, a propargyl ether group, and groups represented by Formulae (r-1) to (r-12).
(7) The thermosetting compound according to any one of (1) to (5), wherein the R¹-D¹ group and the R²-D² group in Formula (1) are each, identically or differently, a group selected from the groups represented by Formulae (r-1) to (r-12).
(8) The thermosetting compound according to any one of (1) to (7), wherein the thermosetting compound has a molecular weight of 748 or more (preferably 748 to 1300, more preferably 748 to 1200, particularly preferably 748 to 1000, and most preferably 748 to 900).
(9) The thermosetting compound according to any one of (1) to (8), wherein the thermosetting compound has solubility in a solvent selected from the class consisting of acetone, methyl ethyl ketone, methyl isobutyl ketone, acetonylacetone, cyclohexanone, isophorone, 2-heptanone, 3-heptanone, benzene, toluene, xylene, dichloromethane, chloroform, 1,2-dichloroethane, chlorobenzene, dichlorobenzene, benzotrifluoride, acetonitrile, benzonitrile, ethyl acetate, tetrahydrofuran, N-methyl-2-pyrrolidone, N,N-dimethylformamide, dimethyl sulfoxide, and mixtures of them.
(10) The thermosetting compound according to any one of (1) to (9), wherein the thermosetting compound has thermotropic liquid crystallinity.
(11) The thermosetting compound according to any one of (1) to (10), wherein the thermosetting compound has a melting point of 300°C or lower (preferably 100°C to 300°C, more preferably 120°C to 290°C, and particularly preferably 150°C to 280°C).
(12) The thermosetting compound according to any one of (1) to (11), wherein the thermosetting compound has a 5% weight loss temperature of 300°C or higher (preferably 330°C or higher, and particularly preferably 350°C or higher) as measured in nitrogen at a rate of temperature rise of 10°C/min.
(13) The thermosetting compound according to any one of (1) to (11), wherein the thermosetting compound has a 5% weight loss temperature of 300°C to 400°C (preferably 330°C to 400°C, and particularly preferably 350°C to 400°C) as measured in nitrogen at a rate of temperature rise of 10°C/min.
(14) The thermosetting compound according to any one of (1) to (13), wherein the thermosetting compound has an exothermic peak temperature of 200°C to 350°C (preferably 250°C to 330°C, and particularly preferably 280°C to 340°C).
(15) A thermosetting composition including the thermosetting compound according to any one of (1) to (14).
(16) The thermosetting composition according to (15), wherein the thermosetting compound is present in a content of 30 weight percent or more (preferably 50 weight percent or more, particularly preferably 70 weight percent or more, and most preferably 90 weight percent or more) of the totality of the thermosetting composition, where, when the thermosetting composition contains two or more of the thermosetting compounds, the term "content" is read as the total content of them, and wherein the thermosetting compound represented by Formula (1) is present in a proportion of 70 weight percent or more (preferably 80 weight percent or more, particularly preferably 90 weight percent or more) of the totality of all curable compound(s) contained in the thermosetting composition.
(17) The thermosetting composition according to one of (15) and (16), wherein the thermosetting composition has a total content of a crosslinker and a curing accelerator of 3 weight percent or less (and preferably less than 1 weight percent), of the totality of the thermosetting composition.
(18) The thermosetting composition according to any one of (15) to (17), wherein the thermosetting composition is one selected from the class consisting of encapsulating agents, coating agents, adhesives, inks, sealants, resists, and lens-forming materials.
(19) A cured product of the thermosetting composition according to any one of (15) to (18).
(20) The cured product according to (19), wherein the cured product has a 5% weight loss temperature of 400°C or higher (preferably 430°C or higher, and particularly preferably 450°C or higher) as measured in nitrogen at a rate of temperature rise of 10°C/min.
(21) The cured product according to (19), wherein the cured product has a 5% weight loss temperature of 400°C to 500°C (preferably 430°C to 500°C, and particularly preferably 450°C to 500°C) as measured in nitrogen at a rate of temperature rise of 10°C/min.
(22) The cured product according to any one of (19) to (21), wherein the cured product has a glass transition temperature (Tg) of higher than 350°C (preferably 380°C or higher, and particularly preferably 400°C or higher).
(23) The cured product according to any one of (19) to (22), wherein the cured product has a storage modulus (E') of 1 GPa or more (preferably 1.1 GPa or more, and particularly preferably 1.15 GPa or more) at 250°C.
(24) The cured product according to any one of (19) to (22), wherein the cured product has a storage modulus (E') of 1 to 2 GPa (preferably 1.1 to 2 GPa, and particularly preferably 1.15 to 2 GPa) at 250°C.
(25) A method for producing a semiconductor device, the method including the step of encapsulating a semiconductor element using the thermosetting composition according to any one of (15) to (18).
(26) A semiconductor device structurally including a semiconductor element, and the cured product according to any one of (19) to (24), the cured product covering the semiconductor element.

### Advantageous Effects of Invention

The thermosetting compounds according to the present invention, which have the configurations, have satisfactory solvent solubility. The thermosetting compounds can be rapidly cured by a heat treatment to give cured products having extreme heat resistance (specifically, having a 5% weight loss temperature (T_{d5}) of 450°C or higher and a storage modulus (E') of 1 GPa or more at 250°C). The thermosetting compounds according to the present invention are therefore advantageously usable typically as or for encapsulating agents for semiconductor devices.

### Description of Embodiments

### Thermosetting Compounds

The thermosetting compounds according to the present invention are represented by Formula (1):

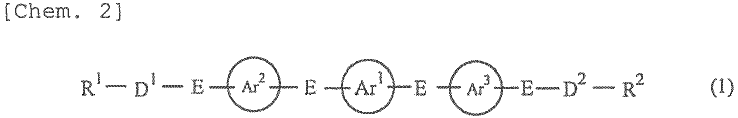

In Formula (1), R¹ and R² each independently represent a thermosetting group; D¹ and D² are each, identically or differently, selected from a single bond and a linkage group; Ar¹, Ar², and Ar³ are each, identically or differently, selected from a divalent aromatic hydrocarbon group and a divalent group including two or more aromatic hydrocarbons bonded to each other via any one selected from a single bond, a divalent aliphatic hydrocarbon group, and a divalent alicyclic hydrocarbon group; and E represents, identically or differently on each occurrence, an ester bond selected from -(C=O)O- and -O(C=O)-.

The divalent aromatic hydrocarbon group as Ar¹, Ar², and Ar³ is a group resulting from removing two hydrogen atoms from the structural formula of an aromatic hydrocarbon. Non-limiting examples of the aromatic hydrocarbon include C₆-C₁₄ aromatic hydrocarbons such as benzene, naphthalene, anthracene, and phenanthrene.

Non-limiting examples of the divalent aliphatic hydrocarbon group include C₁-C₁₈ linear or branched alkylenes and C₂-C₁₈ linear or branched alkenylenes. Non-limiting examples of the C₁-C₁₈ linear or branched alkylenes include methylene, methylmethylene, dimethylmethylene, ethylene, propylene, and trimethylene. Non-limiting examples of the C₂-C₁₈ linear or branched alkenylenes include vinylene, 1-methylvinylene, propenylene, 1-butenylene, and 2-butenylene.

Non-limiting examples of the divalent alicyclic hydrocarbon group include C₃-C₁₈ divalent alicyclic hydrocarbon groups exemplified by cycloalkylenes (including cycloalkylidenes), such as 1,2-cyclopentylene, 1,3-cyclopentylene, cyclopentylidene, 1,2-cyclohexylene, 1,3-cyclohexylene, 1,4-cyclohexylene, and cyclohexylidene.

Ar¹, Ar², and Ar³ are each preferably, identically or differently, a group selected from groups represented by Formulae (a1) to (a4) as follows. The bonds indicated in the formulae are not limited in their bonding positions.

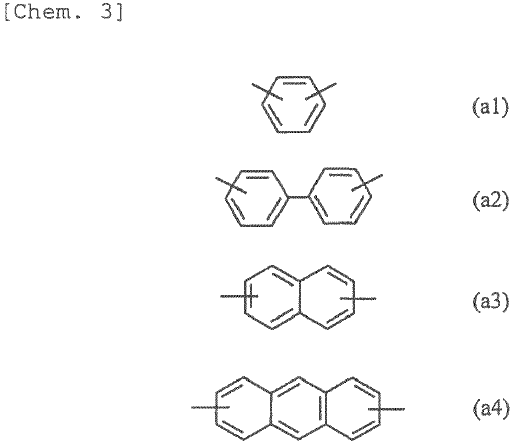

Non-limiting examples of the thermosetting group as R¹ and R² include phenylethynyl, styryl, maleimidyl, nadimidyl, biphenylenyl, ethynyl, isocyanato, cyanato, nitrile, phthalonitrile, cyclobenzobutenyl, benzoxazinyl, oxetanyl, and vinyl. R¹ and R² may present identical groups or different groups.

In particular, the thermosetting group as R¹ and R² is preferably a thermosetting group selected from the class consisting of phenylethynyl, styryl, maleimidyl, nadimidyl, biphenylenyl, phthalonitrile, cyclobenzobutenyl, and benzoxazinyl. These groups are preferred for allowing the thermosetting compounds to have thermotropic liquid crystallinity and to give cured products having particularly excellent heat resistance.

Non-limiting examples of the linkage group as D¹ and D² include divalent hydrocarbon groups, divalent heterocyclic groups, carbonyls, ether bonds, ester bonds, carbonate bonds, amide bonds, imide bonds, and groups each including two or more of them linked to each other.

The category "divalent hydrocarbon group" includes divalent aliphatic hydrocarbon groups, divalent alicyclic hydrocarbon groups, and divalent aromatic hydrocarbon groups, each of which is exemplified as with above.

Heterocycles constituting the divalent heterocyclic groups include aromatic heterocycles and non-aromatic heterocycles. Non-limiting examples of such heterocycles include 3- to 10-membered rings (preferably 4- to 6-membered rings) each containing, as atoms constituting the ring, one or more carbon atoms and at least one heteroatom (such as oxygen atom, sulfur atom, or nitrogen atom); and fused rings derived from these rings. Specifically, non-limiting examples of the heterocycles include oxygen-containing heterocycles, sulfur-containing heterocycles, and nitrogen-containing heterocycles, where oxygen, sulfur, and nitrogen atoms are contained as heteroatoms. Non-limiting examples of the oxygen-containing heterocycles include 3-membered rings such as oxirane ring; 4-membered rings such as oxetane ring; 5-membered rings such as furan, tetrahydrofuran, oxazole, isoxazole, and γ-butyrolactone rings; 6-membered rings such as 4-oxo-4H-pyran, tetrahydropyran, and morpholine rings; fused rings such as benzofuran, isobenzofuran, 4-oxo-4H-chromene, chroman, and isochroman rings; and bridged rings such as 3-oxatricyclo[4.3.1.1^{4,8}]undecan-2-one and 3-oxatricyclo[4.2.1.0^{4,8}]nonan-2-one rings. Non-limiting examples of the sulfur-containing heterocycles include 5-membered rings such as thiophene, thiazole, isothiazole, and thiadiazole rings; 6-membered rings such as 4-oxo-4H-thiopyran ring; and fused rings such as benzothiophene ring. Non-limiting examples of the nitrogen-containing heterocycles include 5-membered rings such as pyrrole, pyrrolidine, pyrazole, imidazole, and triazole rings; 6-membered rings such as isocyanurate, pyridine, pyridazine, pyrimidine, pyrazine, piperidine, and piperazine rings; and fused rings such as indole, indoline, quinoline, acridine, naphthyridine, quinazoline, and purine rings. The divalent heterocyclic groups are groups resulting from removing each two hydrogen atoms from the structural formulae of the heterocycles.

In particular, D¹ and D² are each preferably selected from a single bond and a divalent hydrocarbon group, for allowing the thermosetting compounds to give cured products having particularly excellent heat resistance. Of such divalent hydrocarbon groups, divalent aromatic hydrocarbon groups are preferred, C₆-C₁₄ divalent aromatic hydrocarbon groups are particularly preferred, and the groups represented by Formulae (a1) to (a4) are most preferred.

Accordingly, the R¹-D¹ group and R²-D² group in Formula (1) are preferably each, identically or differently, a group selected from nadimidyl, biphenylenyl, ethynyl, isocyanato, cyanato, nitrile, phthalonitrile, cyclobenzobutenyl, benzoxazinyl, oxetanyl, vinyl, methylmaleimidyl, cinnamoyl, a propargyl ether group, and groups represented by Formulae (r-1) to (r-12) as follows; and are particularly preferably each, identically or differently, a group selected from the groups represented by Formulae (r-1) to (r-12):

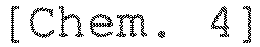

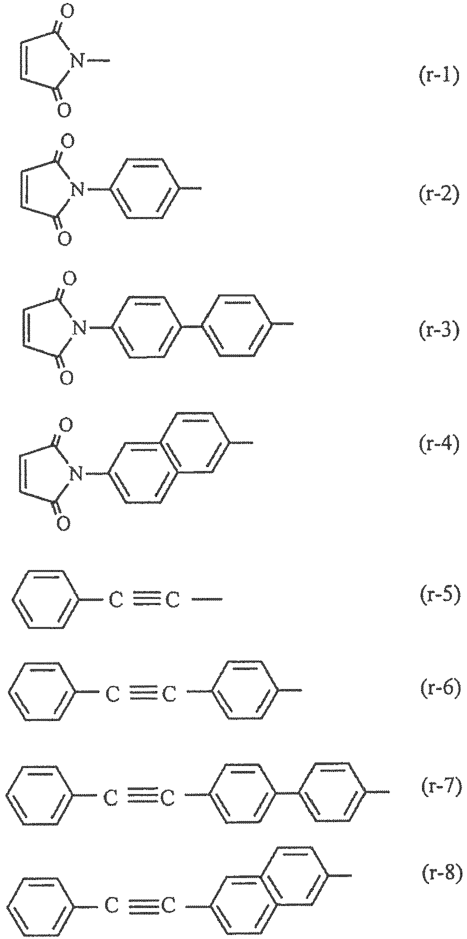

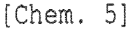

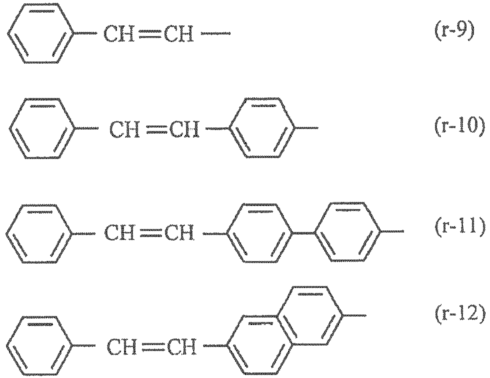

The aromatic hydrocarbon rings of the thermosetting compounds represented by Formula (1) may each have, singly or multiply, one or more types of substituents. Non-limiting examples of the substituents include C₁-C₆ alkyls, C₆-C₁₀ aryls, C₁-C₆ alkoxys, C₆-C₁₀ aryloxys, and halogens.

Non-limiting examples of the C₁-C₆ alkyls include linear or branched alkyls such as methyl, ethyl, propyl, isopropyl, butyl, isobutyl, s-butyl, t-butyl, pentyl, and hexyl.

Non-limiting examples of the C₆-C₁₀ aryls include phenyl and naphthyl.

Non-limiting examples of the C₁-C₆ alkoxys include linear or branched alkoxys such as methoxy, ethoxy, butoxy, and t-butyloxy.

Non-limiting examples of the C₆-C₁₀ aryloxys include phenyloxy and 2-naphthyloxy.

The thermosetting compounds represented by Formula (1) have molecular weights of 748 or more. The thermosetting compounds therefore give, as a result of thermal curing, cured products having extreme heat resistance (specifically, having a 5% weight loss temperature (T_{d5}) of 450°C or higher and a storage modulus (E') of 1 GPa or more at 250°C). In contrast, the thermosetting compounds, if having molecular weights less than the level, tend to fail to give cured products having extreme heat resistance. The molecular weights of the thermosetting compounds may be determined typically by GPC measurement, HPLC measurement, or NMR measurement.

The upper limit of the molecular weights of the thermosetting compounds represented by Formula (1) is typically 1300, preferably 1200, particularly preferably 1000, and most preferably 900. The thermosetting compounds preferably have molecular weights of 1300 or less, because these thermosetting compounds melt at a relatively low temperature, have particularly excellent solubility in a solvent, and are rapidly curable. Non-limiting examples of the solvent include ketones such as acetone, methyl ethyl ketone, methyl isobutyl ketone, acetonylacetone, cyclohexanone, isophorone, 2-heptanone, and 3-heptanone; aromatic hydrocarbons such as benzene, toluene, and xylenes; halogenated hydrocarbons such as dichloromethane, chloroform, 1,2-dichloroethane, chlorobenzene, dichlorobenzene, and benzotrifluoride; nitriles such as acetonitrile and benzonitrile; esters such as ethyl acetate; ethers such as tetrahydrofuran; N-methyl-2-pyrrolidone; N,N-dimethylformamide; dimethyl sulfoxide; and mixtures of them.

Of the thermosetting compounds represented by Formula (1), compounds in which the R¹-D¹ group and R²-D² group are each the group represented by Formula (r-2) can be produced typically through the steps (1) and (2) as follows. Of the thermosetting compounds represented by Formula (1), compounds in which the R¹-D¹ group and R²-D² group are each independently a group other than the group represented by Formula (r-2) can be produced by a procedure similar to or in accordance with the production method as follows.

In the step (1), each one molecule of an aromatic diol, an aromatic dicarboxylic acid, and an aromatic hydroxycarboxylic acid as reaction substrates is reacted (esterified) with one another, or one molecule of a compound selected from the class consisting of an aromatic diol, an aromatic dicarboxylic acid, and an aromatic hydroxycarboxylic acid is reacted (esterified) with two molecules of a compound selected from the remainder of the class, to give a carboxylic ester (trimer) containing at least one of hydroxy and carboxy at both ends.

In the step (2), the carboxylic ester (trimer) containing at least one of hydroxy and carboxy at both ends is reacted (esterified) with a N-phenylmaleimide containing a functional group reactive with the at least one of hydroxy and carboxy, to give the thermosetting compound represented by Formula (1).

Of the thermosetting compounds represented by Formula (1), the compounds where the R¹-D¹ group and R²-D² group are each the group represented by Formula (r-2) may be produced typically by the following method, which is illustrated as an example. In the formula, Ar¹ and Ar² are as defined above and may be identical to or different from each other.

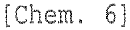

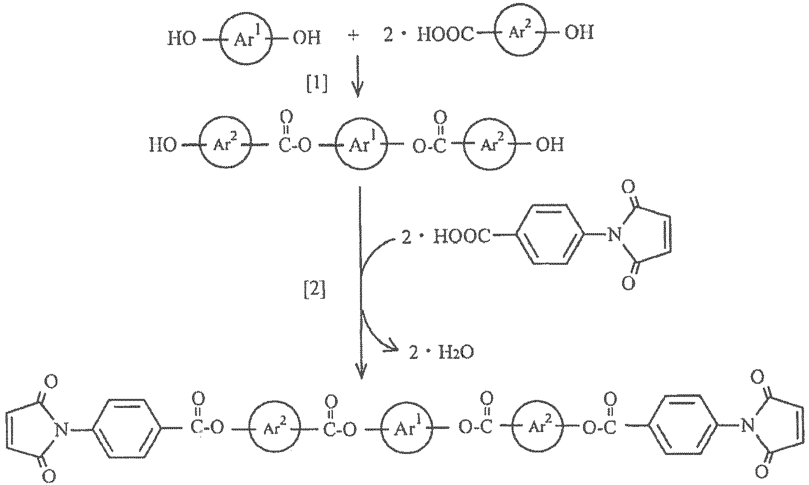

Non-limiting examples of the aromatic diol include hydroquinone, 4,4'-dihydroxybiphenyl, resorcinol, 2,6-naphthalenediol, 1,5-naphthalenediol, (1,1'-biphenyl)-4,4'-diol, 4,4'-dihydroxydiphenyl ether, bis(4-hydroxyphenyl)methanone, bisphenol-A, bisphenol-F, bisphenol-S, (1,1'-biphenyl)-2,5-diol, and derivatives of them. Non-limiting examples of the derivatives include compounds corresponding to the aromatic diols, except with one or more substituents bonded to the aromatic hydrocarbon groups of the aromatic diols. Examples of the substituents are as with the substituents which the aromatic hydrocarbon rings may have.

Non-limiting examples of the aromatic dicarboxylic acid include phthalic acid, terephthalic acid, isophthalic acid, 2,6-naphthalenedicarboxylic acid, 1,5-naphthalenedicarboxylic acid, 4,4'-dibenzoic acid, 4,4'-oxybisbenzoic acid, 4,4'-thiodibenzoic acid, 4-[2-(4-carboxyphenoxy)ethoxy]benzoic acid, and derivatives of them. Non-limiting examples of the derivatives include compounds corresponding to the aromatic dicarboxylic acids, except with one or more substituents bonded to the aromatic hydrocarbon groups of the aromatic dicarboxylic acids. Examples of the substituents are as with the substituents which the aromatic hydrocarbon rings may have.

Non-limiting examples of the aromatic hydroxycarboxylic acid include 4-hydroxybenzoic acid, 3-hydroxybenzoic acid, 1-hydroxy-2-naphthoic acid, 3-hydroxy-2-naphthoic acid, 6-hydroxy-2-naphthoic acid, 5-hydroxy-1-naphthoic acid, 4'-hydroxy (1,1'-biphenyl)-4-carboxylic acid, and derivatives of them. Non-limiting examples of the derivatives include compounds corresponding to the aromatic hydroxycarboxylic acids, except with one or more substituents bonded to the aromatic hydrocarbon groups of the aromatic hydroxycarboxylic acids. Examples of the substituents are as with the substituents which the aromatic hydrocarbon rings may have.

In the N-phenylmaleimide having a functional group reactive with at least one of hydroxy and carboxy, the functional group reactive with hydroxy is preferably carboxy; and the functional group reactive with carboxy is preferably hydroxy. Accordingly, the N-phenylmaleimide having a functional group reactive with hydroxy is preferably N-(4-carboxyphenyl)maleimide; and the N-phenylmaleimide having a functional group reactive with carboxy is preferably N-(4-hydroxyphenyl)maleimide.

The esterification in the steps (1) and (2) may be performed typically by (i) a process of performing esterification in the presence of a catalyst, or by (ii) a process of performing esterification in the presence of a condensing agent, or by (iii) a process of halogenating carboxy, and performing esterification after the halogenation.

The catalyst for use in the process (i) may be any of protonic acids and Lewis acids. Non-limiting examples of the protonic acids include organic acids and inorganic acids, exemplified by super strong acids (such as SbF₅, SbF₅-HF, SbF₅-FSO₃H, and SbF₅-CF₃SO₃H), sulfuric acid, hydrochloric acid, phosphoric acid, fluoroboric acid, p-toluenesulfonic acid, chloroacetic acid, picric acid, and heteropolyacids. Non-limiting examples of the Lewis acids include B(OH)₃, BF₃, BF₃O(C₂H₅)₂, AlCl₃, and FeCl₃. Each of them may be used alone or in combination.

The catalyst(s) may be used in an amount of typically 1.0 to 50.0 mole percent, relative to the totality (total moles) of the reaction substrates. When two or more catalysts are used, the term "amount" is read as the total amount of them.

Non-limiting examples of the condensing agent in the process (ii) include carbodiimide condensing agents such as 1-ethyl-3-(3-dimethylaminopropyl)carbodiimide hydrochloride, 1-[3-(dimethylamino)propyl]-3-ethylcarbodiimide, N,N'-dicyclohexylcarbodiimide, N,N'-diisopropylcarbodiimide, and N-cyclohexyl-N'-(2-morpholinoethyl)carbodiimide p-toluenesulfonate; imidazole condensing agents such as N,N'-carbonyldiimidazole; as well as 4-(4,6-dimethoxy-1,3,5-triazin-2-yl)-4-methylmorpholinium chloride n-hydrate, and (4,6-dimethoxy-1,3,5-triazin-2-yl)-(2-octoxy-2-oxoethyl)dimethylammonium trifluoromethanesulfonate. Each of them may be used alone or in combination.

The condensing agent(s) may be used in an amount of typically 100 to 300 mole percent relative to the totality (total moles) of the reaction substrates. When two or more different condensing agents are used, the term "amount" is read as the total amount of them.

Non-limiting examples of the halogenating agent used for halogenation of carboxy in the process (iii) include thionyl chloride, oxalyl chloride, phosphorus pentachloride, phosphorus trichloride, thionyl bromide, and phosphorus tribromide. Each of them may be used alone or in combination.

The halogenating agent(s) may be used in an amount of typically 1.0 to 3.0 moles per mole of carboxy.

In the process (iii), a hydrogen halide is formed with the progress of esterification. The esterification is therefore preferably performed in the presence of a base for trapping the formed hydrogen halide. This is preferred for effectively allowing the esterification to proceed. Non-limiting examples of the base include inorganic bases such as sodium hydroxide, potassium hydroxide, calcium hydroxide, sodium carbonate, potassium carbonate, and sodium hydrogencarbonate; and organic bases such as pyridine and triethylamine. Each of them may be used alone or in combination.

The base(s) may be used in an amount of typically about 1.0 to about 3.0 moles per mole of acyl halide groups in the halide of the carboxylic acid.

The esterification in the steps (1) and (2) may be performed in the presence of a solvent. Non-limiting examples of the solvent include ketones such as acetone, methyl ethyl ketone, methyl isobutyl ketone, acetonylacetone, cyclohexanone, isophorone, 2-heptanone, and 3-heptanone; aromatic hydrocarbons such as benzene, toluene, and xylenes; halogenated hydrocarbons such as dichloromethane, chloroform, 1,2-dichloroethane, dichlorobenzene, and benzotrifluoride; and nitriles such as acetonitrile and benzonitrile. Each of them may be used alone or in combination.

The solvent(s) may be used in an amount of typically about 5 to about 20 times by weight the totality (total weight) of the reaction substrates. The solvent(s), if used in an amount greater than the range, tends to cause a lower reaction rate because of lower concentrations of the reaction components.

The reaction atmosphere for the esterification is not limited, as long as not adversely affecting the reaction, and may be any atmosphere such as air atmosphere, nitrogen atmosphere, or argon atmosphere.

The esterification in the steps (1) and (2) may be performed at a reaction temperature of typically about 0.0°C to about 200.0°C for a reaction time of typically about 0.5 to about 3 hours. The esterification may be performed according to any system such as batch system, semi-batch system, or continuous system.

After the completion of the esterification in the steps (1) and (2), the resulting reaction product can be separated and purified typically by a separation means such as filtration, concentration (thickening), distillation, extraction, crystallization, adsorption, recrystallization, or column chromatography, or a separation means as any combination of them.

The thermosetting compounds according to the present invention have thermotropic liquid crystallinity. The thermosetting compounds according to the present invention can be thermally cured in a highly oriented state to form cured products having anisotropy. The resulting cured products therefore have extreme heat resistance. The thermotropic liquid crystallinity of the thermosetting compounds according to the present invention, and the anisotropy of the cured products can be verified by observation with a polarizing microscope.

The thermosetting compounds represented by Formula (1) have melting points (Tm) of typically 300°C or lower (for example, 100°C to 300°C, preferably 120°C to 290°C, and particularly preferably 150°C to 280°C). The thermosetting compounds are therefore meltable at a relatively low temperature and offer excellent workability. The melting points can be measured typically by a thermal analysis such as DSC or TGA, or by dynamic viscoelastic measurement.

The thermosetting compounds represented by Formula (1) have 5% weight loss temperatures (T_{d5}) of typically 300°C or higher (for example, 300°C to 400°C), more preferably 330°C or higher, and particularly preferably 350°C or higher, as measured at a rate of temperature rise of 10°C/min (in nitrogen). The "5% weight loss temperature" in this description may be measured typically by simultaneous thermogravimetry/differential thermal analysis (TG/DTA).

The thermosetting compounds represented by Formula (1) have exothermic peak temperatures of typically 200°C to 350°C (preferably 250°C to 330°C, and particularly preferably 280°C to 340°C). The exothermic peak temperatures can be measured typically by thermal analysis through DSC.

Accordingly, the thermosetting compounds represented by Formula (1) can be rapidly thermally cured to form cured products having extreme heat resistance, by heating at a temperature of typically 200°C to 350°C (preferably 250°C to 330°C, and particularly preferably 280°C to 340°C) for typically 10 to 120 minutes (preferably 10 to 60 minutes, and particularly preferably 10 to 30 minutes). The heating may be performed at a temperature held constant, or at temperatures varying stepwise, within the temperature range. The heating temperature is preferably adjusted within the range as appropriate according to the heating time. For example, when a shorter heating time is desired, the heating temperature is preferably set on the high side. The thermosetting compounds according to the present invention have the structures represented by Formula (1) and can form cured products (specifically, cured products having extreme heat resistance) without decomposing by heating even at a high temperature and can efficiently form the cured products with excellent workability, by heating at a high temperature for a short time. The heating means is not particularly limited and may be selected from among known or common means.

The curing of the thermosetting compounds represented by Formula (1) may be performed at normal atmospheric pressure, under reduced pressure, or under pressure (under a load).

The cured products of the thermosetting compounds represented by Formula (1) have 5% weight loss temperatures (T_{d5}) of typically 400°C or higher (for example, 400°C to 500°C), more preferably 430°C or higher, and particularly preferably 450°C or higher, as measured at a rate of temperature rise of 10°C/min (in nitrogen).

The cured products of the thermosetting compounds represented by Formula (1) may each have a glass transition point, or not. Even when the cured products have a glass transition point, no glass transition point is detected within the temperature range typically up to 350°C (preferably up to 380°C, and particularly preferably up to 400°C) in DSC measurement. Accordingly, the cured products of the thermosetting compounds represented by Formula (1), when having a glass transition point, have glass transition temperatures (Tg) of typically higher than 350°C (preferably 380°C or higher, and particularly preferably 400°C or higher).

In addition, the cured products of the thermosetting compounds represented by Formula (1) have high hardness even in a high-temperature environment and have storage moduli (E') of typically 1 GPa or more (for example, 1 to 2 GPa), preferably 1.1 GPa or more, and particularly preferably 1.15 GPa or more, at 250°C.

The thermosetting compounds according to the present invention have satisfactory solvent solubility (are satisfactorily soluble in a solvent or solvents). The thermosetting compounds can be rapidly cured by a heat treatment to form cured products having extreme heat resistance, as described above. Thus, the thermosetting compounds are preferably usable typically as or for encapsulating agents, coating agents, adhesives, inks, sealants, resists, and forming materials. Non-limiting examples of the forming materials include materials for the formation typically of substrates, electrical insulating materials (such as insulating films), laminated sheets, composite materials (such as fiber-reinforced plastics), optical elements (such as lenses), stereolithographic materials, electronic papers, touch screens (touch panels), solar cell substrates, optical waveguides, light guide plates, and holographic memories. In particular, the thermosetting compounds are preferably usable as or for encapsulating agents to cover or encapsulate semiconductor elements in high heat resistance-high breakdown voltage semiconductor devices (such as power semiconductor devices), where conventional resinous materials are hardly provided for these uses.

### Thermosetting Compositions

The thermosetting compositions according to the present invention contain each of the thermosetting compounds alone or in combination. The thermosetting compositions according to the present invention may contain the thermosetting compound in a content of typically 30 weight percent or more, preferably 50 weight percent or more, particularly preferably 70 weight percent or more, and most preferably 90 weight percent or more, of the totality of the composition. When the composition contains two or more of the thermosetting compounds, the term "content" is read as the total content of them. The upper limit of the content is 100 weight percent. Namely, the thermosetting compositions according to the present invention include ones containing the thermosetting compound or compounds alone.

The thermosetting compositions according to the present invention may further contain one or more other components as needed, in addition to the thermosetting compound or compounds. The other components for use herein may be selected typically from known or common additives. Non-limiting examples of such other components include curable compounds other than the compounds represented by Formula (1), catalysts, fillers, organic resins (such as silicone resins, epoxy resins, and fluorocarbon resins), solvents, stabilizers (such as antioxidants, ultraviolet absorbers, photostabilizers, and thermal stabilizers), flame retardants (such as phosphorus flame retardants, halogen flame retardants, and inorganic flame retardants), flame retardant promoters, reinforcers, nucleating agents, coupling agents, lubricants, waxes, plasticizers, release agents, impact modifiers, color modifiers, flow improvers, colorants (such as dyes and pigments), dispersants, antifoaming agents, defoaming agents, antimicrobial agents, antiseptic agents, viscosity modifiers, and thickeners. The thermosetting compositions may contain each of them alone or in combination.

The thermosetting compositions according to the present invention may further contain a curable compound other than the thermosetting compounds represented by Formula (1). However, the thermosetting compound or compounds represented by Formula (1) are present in a proportion of typically 70 weight percent or more, preferably 80 weight percent or more, and particularly preferably 90 weight percent or more, of the totality of all curable compounds contained in the thermosetting composition. The upper limit of the proportion is 100 weight percent.

The thermosetting compositions according to the present invention can rapidly form cured products, even when containing neither a crosslinker nor a curing accelerator (for example, even when the thermosetting compositions according to the present invention have a total content of a crosslinker and a curing accelerator of typically 3 weight percent or less, and preferably less than 1 weight percent). The resulting cured products therefore have high 5% weight loss temperatures (T_{d5}) and offer extreme heat resistance. In addition, the cured products can have extremely low contents of an unreacted curing accelerator and decomposed products of the curing accelerator, and less undergo the formation of outgases derived from these substances.

The thermosetting compositions according to the present invention contain the thermosetting compound or compounds and can be rapidly cured by a heat treatment to form cured products having extreme heat resistance. The conditions for the heat treatment may be set as appropriate within ranges similar to the ranges of the conditions for thermal curing of the thermosetting compounds.

The thermosetting compositions according to the present invention are preferably usable as encapsulating agents, coating agents, adhesives, inks, sealants, resists, and forming materials. Non-limiting examples of the forming materials are those for substrates, electrical insulating materials (such as insulating films), laminated sheets, composite materials (such as fiber-reinforced plastics), optical elements (such as lenses), stereolithographic materials, electronic papers, touch screens (touch panels), solar cell substrates, optical waveguides, light guide plates, and holographic memories. In particular, the thermosetting compositions are preferably usable as encapsulating agents to cover or encapsulate semiconductor elements in high heat resistance-high breakdown voltage semiconductor devices (such as power semiconductor devices), where conventional resinous materials are hardly provided for these uses.

### Semiconductor Device Production Method, and Semiconductor Device Produced by The Method

The method according to the present invention for producing a semiconductor device includes the step of encapsulating a semiconductor element using any of the thermosetting compositions (for example, using the thermosetting composition as a semiconductor encapsulating agent).

The encapsulation of the semiconductor element may be performed according to a known or common procedure without limitation, and may be performed typically by injecting the thermosetting composition according to the present invention into a predetermined forming mold, and subjecting the composition in the mold to a heat treatment. The conditions for the heat treatment may be set as appropriate within ranges similar to the ranges of the conditions for thermal curing of the thermosetting compounds.

The resulting semiconductor device obtained by the method according to the present invention for producing a semiconductor device is a semiconductor device that includes a semiconductor element, and an encapsulant covering the semiconductor element, where the encapsulant is the cured product of any of the thermosetting compositions according to the present invention.

The semiconductor device according to the present invention includes a semiconductor element encapsulated using the thermosetting composition according to the present invention. This enables encapsulation of the semiconductor element with protecting the semiconductor element and surrounding structures from deteriorating by heat upon curing. In addition, the semiconductor element is protected by the cured product that is tough even in a high-temperature environment, because the cured product (i.e., encapsulant) of the thermosetting composition has extreme heat resistance. Accordingly, the semiconductor device obtained by the production method according to the present invention has high functions and offers high reliability. Examples

The present invention will be illustrated in further detail with reference to several examples below. It should be noted, however, that the examples are by no means intended to limit the scope of the present invention.

The NMR measurement was performed under conditions as follows.

A nuclear magnetic resonance (NMR) measuring apparatus JNM-ECA500 (trade name, supplied by JEOL RESONANCE) was used. Deuterated solvents (described in the examples and the comparative example) were used as measurement solvents, and TMS was used as a chemical shift reference.

### Example 1

### Production of Carboxylic Ester Containing Hydroxys at Both Ends

Materials were placed in a 1.0-L three-necked flask equipped with a stirring bar, a condenser, and a Dean-Stark apparatus, where the materials were 380.0 mL (3.1 mol) of xylene, 20.0 g (181.6 mmol) of hydroquinone, 52.7 g (381.4 mmol) of hydroxybenzoic acid, 0.74 g (12.0 mmol) of boric acid, and 1.9 g (19.0 mmol) of sulfuric acid. The mixture was stirred in a nitrogen atmosphere under reflux for one hour, to complete esterification. The reaction mixture was cooled down to room temperature, from which precipitates were separated, washed with methanol, dried, and yielded 33.9 g (96.7 mmol) of a compound (1) as white crystals. The compound (1) was identified on its chemical structure by NMR measurement and was found to be a compound (1,4-phenylenebis(4-hydroxybenzoate), molecular weight: 350.3) represented by the formula: 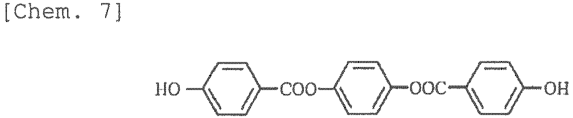 ¹H-NMR (DMSO-d₆)
δ: 6.94 (4H, d, J = 9.0 Hz), 7.32 (4H, s), 8.00 (4H, d, J = 9.0 Hz), 10.53 (2H, s).

### Thermosetting Compound Production

Materials were placed in a 100-mL three-necked flask equipped with a stirring bar and a condenser, where the materials were 23.0 mL (2216.4 mmol) of toluene, 5.0 g (22.8 mmol) of 4-maleimidobenzoic acid, 2.2 mL (30.4 mmol) of thionyl chloride, and 0.36 mL (4.6 mmol) of N,N-dimethylformamide. The resulting mixture was stirred at 80°C in a nitrogen atmosphere for one hour, to complete chlorination. Volatile components were then distilled off under reduced pressure, to leave 4-maleimidobenzoyl chloride as pale yellow crystals. Next, 5.4 g (22.8 mmol) of above-prepared 4-maleimidobenzoyl chloride, 40.0 mL (353.7 mmol) of o-dichlorobenzene, 2.0 g (5.7 mmol) of the compound (1), and 3.2 mL (22.8 mmol) of triethylamine were stirred in a nitrogen atmosphere for one hour with heating at 80°C, to complete esterification. Then the reaction mixture was cooled down to room temperature, from which precipitates were separated, washed with methanol, dried, and yielded 4.1 g (5.5 mmol) of a compound (2) as pale yellow crystals. The above-prepared compound (2) was identified on its chemical structure by NMR measurement and was found to be a compound (molecular weight: 748.7) represented by Formula (1-1) as follows. The compound (2) was found to have thermotropic liquid crystallinity, by observation with a polarizing microscope. In addition, the compound (2) was found to have satisfactory solubility in DMSO, DMF, and NMP. 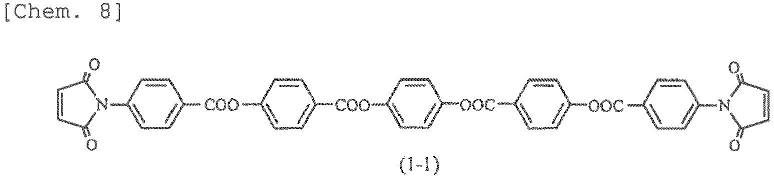 ¹H-NMR (DMSO-d₆)
δ: 7.25 (4H, s), 7.44 (4H, s), 7.58-7.65 (8H, m), 8.28 (8H, d, J = 6.5 Hz).

The compound (2) was placed between a pair of metal sheets, compressively heated at 340°C for 15 minutes through hot pressing, and yielded a homogeneous cured product (1). The prepared cured product (1) was observed with a polarizing microscope and was found to have anisotropy.

### Example 2

### Production of Carboxylic Ester Containing Hydroxys at Both Ends

Materials were placed in a 100-mL three-necked flask equipped with a stirring bar, a condenser, and a Dean-Stark apparatus, where the materials were 22.0 mL (178.2 mol) of xylene, 4.0 g (21.7 mmol) of 4,4'-biphenol, 3.0 g (21.7 mmol) of hydroxybenzoic acid, 0.044 g (0.72 mmol) of boric acid, and 0.95 g (9.7 mmol) of sulfuric acid. The resulting mixture was stirred in a nitrogen atmosphere under reflux for one hour, to complete esterification. The reaction mixture was cooled down to room temperature, from which precipitates were separated, washed with methanol, dried, and yielded 1.7 g (4.0 mmol) of a compound (3) as white crystals. The prepared compound (3) was identified on its chemical structure by NMR measurement and was found to be a compound (1,1'-biphenyl-4,4'-diylbis(4-hydroxybenzoate), molecular weight: 426.42) represented by the formula: 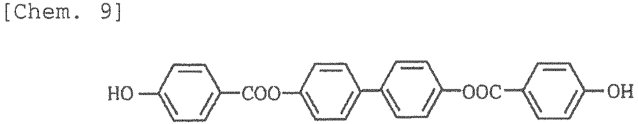 ¹H-NMR (DMSO-d₆)
δ: 6.95 (4H, d, J = 9.5 Hz), 7.35 (4H, d, J = 9.5 Hz), 7.76 (4H, d, J = 9.5 Hz), 8.02 (4H, d, J = 9.5 Hz), 10.55 (2H, s).

### Thermosetting Compound Production

Materials were placed in a 500-mL three-necked flask equipped with a stirring bar and a condenser, where the materials were 31.0 mL (291.7 mmol) of toluene, 6.7 g (31.0 mmol) of 4-maleimidobenzoic acid, 3.0 mL (41.2 mmol) of thionyl chloride, and 0.48 mL (6.2 mmol) of N,N-dimethylformamide. The resulting mixture was stirred at 80°C in a nitrogen atmosphere for one hour, to complete chlorination. Then, volatile components were distilled off under reduced pressure, to leave 4-maleimidobenzoyl chloride as pale yellow crystals. Next, 7.3 g (31.0 mmol) of the prepared 4-maleimidobenzoyl chloride, 135.0 mL (1.2 mol) of o-dichlorobenzene, 6.0 g (14.1 mmol) of the compound (3), and 4.3 mL (31.0 mmol) of triethylamine were stirred in a nitrogen atmosphere with heating at 80°C for one hour, to complete esterification. Then the reaction mixture was cooled down to room temperature, from which precipitates were separated, washed with methanol, dried, and yielded 8.7 g (10.6 mmol) of a compound (4) as pale yellow crystals. The prepared compound (4) was identified on its chemical structure by NMR measurement and was found to be a compound (molecular weight: 824.8) represented by Formula (1-2) as follows. The compound (4) was observed with a polarizing microscope and was found to have thermotropic liquid crystallinity. In addition, the compound (4) was found to have satisfactory solubility in DMSO, DMF, and NMP. 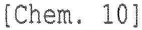 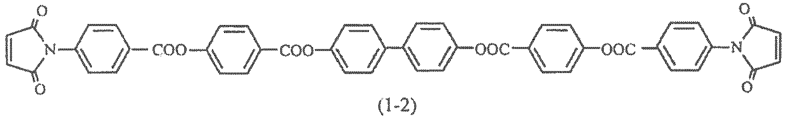 ¹H-NMR (DMSO-d₆)
δ: 7.27 (4H, s), 7.45 (4H, d, J = 8.0 Hz), 7.60 (4H, d, J = 8.5 Hz), 7.66 (4H, d, J = 8.5 Hz), 7.83 (4H, d, J = 8.0 Hz), 8.30 (8H, d, J = 8.5 Hz).

The prepared compound (4) was placed between a pair of metal sheets, compressively heated at 340°C for 15 minutes through hot pressing, and yielded a homogeneous cured product (2). The prepared cured product (2) was observed with a polarizing microscope and was found to have anisotropy.

### Comparative Example 1

### Thermosetting Compound Production

Materials were placed in a 500-mL flask equipped with a condenser and a stirrer, where the materials were 94.3 g (0.682 mol) of 4-hydroxybenzoic acid, 102.7 g (0.546 mol) of 6-hydroxy-2-naphthoic acid, 25.4 g (0.136 mol) of 4,4'-dihydroxybiphenyl, 156.3 g (1.53 mol) of acetic anhydride, and 10.0 mg (0.10 mol) of potassium acetate. The resulting mixture was gradually heated up to 140°C in a nitrogen atmosphere and reacted for 3 hours with the temperature being maintained, to complete acetylation. Next, while the temperature was raised up to 340°C at a rate of 0.8°C/min, acetic acid and unreacted acetic anhydride were distilled off. The inside pressure of the flask was gradually reduced down to 1 Torr to distill off volatile components. This gave a liquid-crystal polyester "a" containing hydroxys at both ends of its molecular chain, where the molecular chain includes aromatic units (constitutional units derived from aromatic compounds) alone. The prepared liquid-crystal polyester "a" was estimated to be a decamer derived from a monomer, as a result of calculation of the number of termini of the liquid-crystal polyester "a" (in accordance with the amine decomposition HPLC method described in Japanese Unexamined Patent Application Publication (JP-A) No. H05-271394), and by GPC measurement.

With 1.42 g of methylenebismaleimide, 3.27 g of the liquid-crystal polyester "a" was melt-blended at 170°C for 6 hours and yielded a reaction product. The reaction product was a composition containing a thermosetting liquid-crystal polyester.

The prepared reaction product was placed between a pair of glass plates, heated up to 240°C using a hot plate, left for 6 hours to allow the curing reaction to proceed, and yielded a homogeneous cured product (3).

The compounds prepared according to the examples and the comparative example were evaluated for melting point, exothermic peak temperature, and 5% weight loss temperature, and the cured products according to the examples and the comparative example were evaluated for 5% weight loss temperature and storage modulus (E') at 250°C, through measurements performed by methods as follows.

### Melting Point and Exothermic Peak Temperature

The compounds (each 5 mg) were heated in a nitrogen stream (50 mL/min) at a temperature of temperature rise of 10°C/min using a differential scanning calorimeter (DSC) DSC 6200 (trade name, supplied by SII NanoTechnology Inc.) to measure the melting point (Tm) and the exothermic peak temperature.

### 5% Weight Loss Temperature (T_{d5})

The compounds or the cured products (about 5 mg) were each heated in a nitrogen stream (300 mL/min) at a temperature of temperature rise of 10°C/min using a thermogravimeter-differential thermal analyzer (TG/DTA) EXSTAR 6300 (trade name, supplied by SII NanoTechnology Inc.) to measure the 5% weight loss temperature (T_{d5}). Alumina was used as a reference material in the measurement.

### Storage Modulus (E') at 250°C

The cured products (about 200 mg) were each subjected to measurement using a dynamic mechanical analyzer (DMA; dynamic viscoelastic analyzer) RSA-III (trade name, supplied by TA Instruments).

The results are given in the following table.

### [Table 1]

**TABLE 1**

| | | Melting point (Tm: °C) | Exothermic peak temperature (°C) | 5% Weight loss temperature (T_{d5}: °C) | Storage modulus (E': GPa) |
|---|---|---|---|---|---|
| Example 1 | Compound (2) | <340* | 320.7 | 325.0 | - |
| | Cured product (1) | - | - | 462.2 | 1.17 |
| Example 2 | Compound (4) | <340* | 299.9 | - | - |
| | Cured product (2) | - | - | 452.9 | 1.46 |
| Comparative Example 1 | Reaction product | 164.1 | - | 69.6 | - |
| | Cured product (3) | - | - | 428 | - |

| | | | | | |
|---|---|---|---|---|---|
| *"< 340" indicates that the sample had been melted at 340°C. | | | | | |

### Industrial Applicability

The thermosetting compounds according to the present invention have satisfactory solvent solubility. The thermosetting compounds can be rapidly cured by a heat treatment to form cured products having extreme heat resistance. The thermosetting compounds according to the present invention are advantageously usable typically as or for encapsulating agents for semiconductor devices.

## Claims

1. A thermosetting compound represented by Formula (1): 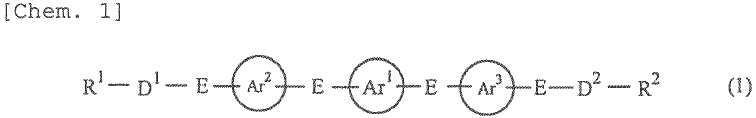 wherein R¹ and R² each independently represent a thermosetting group; D¹ and D² are each, identically or differently, selected from a single bond and a linkage group; Ar¹, Ar², and Ar³ are each, identically or differently, selected from a divalent aromatic hydrocarbon group and a divalent group including two or more aromatic hydrocarbons bonded to each other via any one selected from a single bond, a divalent aliphatic hydrocarbon group, and a divalent alicyclic hydrocarbon group; and E represents, identically or differently on each occurrence, an ester bond selected from -(C=O)O- and -O(C=O)-.

2. The thermosetting compound according to claim 1,
wherein R¹ and R² in Formula (1) are each, identically or differently, a thermosetting group selected from the class consisting of phenylethynyl, styryl, maleimidyl, nadimidyl, biphenylenyl, ethynyl, isocyanato, cyanato, nitrile, phthalonitrile, cyclobenzobutenyl, benzoxazinyl, oxetanyl, and vinyl.

3. The thermosetting compound according to one of claims 1 and 2,
wherein the thermosetting compound has thermotropic liquid crystallinity.

4. A thermosetting composition comprising
the thermosetting compound according to any one of claims 1 to 3.

5. A cured product of the thermosetting composition according to claim 4.

6. A method for producing a semiconductor device, the method comprising the step of
encapsulating a semiconductor element using the thermosetting composition according to claim 4.

7. A semiconductor device structurally comprising:
a semiconductor element; and
the cured product according to claim 5, the cured product covering the semiconductor element.
